# FASCICULE DE BREVET EUROPEEN

(11) **EP 0 408 442 B1**
(45) Date de publication et mention de la délivrance du brevet: **31.08.1994**
(21) Numéro de dépôt: 90401983.3
(22) Date de dépôt: 10.07.1990
(51) Int. Cl.: C07D 239/48, A61K 7/06

(54) **Dérivés de pyrimidine oxyde-3 halogénés, leur utilisation pour le traitement et la prévention de la chute des cheveux et pour stimuler leur repousse**
Halogenierte Pyrimidin-3-oxid-Derivate, deren Verwendung für die Behandlung und die Verhütung von Haarausfall und für die Wachstumsförderung von Haaren
Halogenated pyrimidin-3-oxyde derivatives, their use for the treatment and the prevention of hairloss and for stimulating growth again

(30) Priorité: 12.07.1989 EP 89402002; 31.01.1990 FR 9001148
(43) Date de publication de la demande: 16.01.1991
(73) Titulaire: L'OREAL, 75008 Paris (FR)
(72) Inventeur: Tuloup, Rémy, F-35190 Miniac-sous-Bécherel (FR); Junino, Alex, F-93180 Livry-Gargan (FR); Hocquaux, Michel, F-75012 Paris (FR); Dumats, Jacqueline, F-93420 Villepinte (FR); Gaetani, Quintino, F-93270 Sevran (FR)
(74) Mandataire: Casalonga, Axel

(56) Documents cités:
- EP-A- 0 356 271
- WO-A-86/00616
- DE-A- 3 717 480

## Description

La présente invention est relative à de nouveaux dérivés de pyrimidine oxyde-3 halogénés, à la préparation de compositions cosmétiques ou pharmaceutiques destinées notamment à être utilisées en application topique dans le traitement et la prévention de la chute des cheveux et pour stimuler leur repousse.

On connaît déjà dans le brevet US 4 139 6 19 le pipéridino-6 diamino-2,4 pyrimidine oxyde-3 ou "Minoxidil" et ses dérivés pour leur utilisation dans le traitement de la chute des cheveux, de la pelade, de la dermatite desquamante, de l'alopécie, etc.

La demanderesse vient de découvrir de nouveaux produits dérivés de la pyrimidine oxyde-3, substitués en position 6 par un groupement haloalcoxy.

Elle a découvert que ces produits étaient particulièrement efficaces pour le traitement de la repousse des cheveux et en particulier pour induire et stimuler la croissance des cheveux et freiner leur chute et qu'ils pouvaient notamment être utilisés dans le traitement des maladies du cuir chevelu, telles que la pelade, la dermatite desquamante ou encore l'alopécie.

Un autre avantage de ces composés est leur solubilité remarquable dans des milieux utilisés pour l'application topique.

L'invention a donc pour objet de nouveaux dérivés de pyrimidine oxyde-3 substitués en position 6 par un groupement haloalcoxy.

Un autre objet de l'invention est constitué par leur procédé de préparation.

L'invention concerne également des compositions cosmétiques et/ou pharmaceutiques permettant la mise en oeuvre de ces composés.

D'autres objets de l'invention apparaîtront à la lecture de la description et des exemples qui suivent.

Les composés conformes à l'invention sont essentiellement caractérisés par le fait qu'ils répondent à la formule : 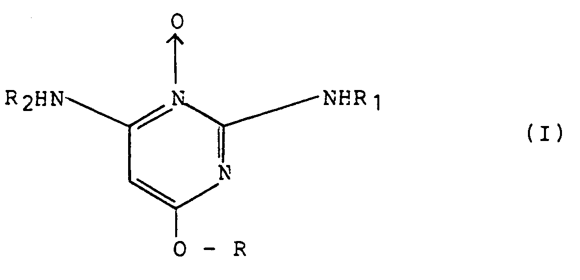
dans laquelle :
R₁ et R₂ désignent, indépendamment l'un de l'autre, un atome d'hydrogène, un groupement carbamoyle de formule : 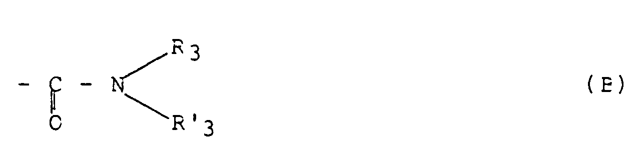
avec R′₃ désignant hydrogène ou R₃;
un groupement alcoxycarbonyle de formule : 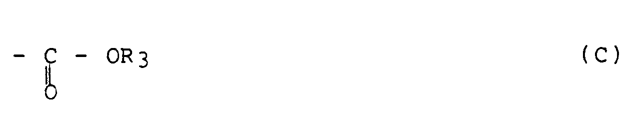
ou un groupement acyle de formule : 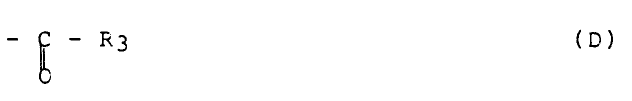
dans lesquels R₃ désigne un radical alkyle linéaire ou ramifié en C₁-C₁₈, un groupement alcényle en C₂-C₁₈, un groupement cycloalkyle en C₅-C₈; R₃ peut également désigner un radical aryle ou aralkyle répondant à la formule : 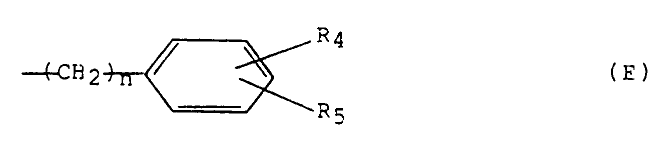
dans laquelle :
n est un nombre entier pouvant varier entre 0 et 4;
R₄ et/ou R₅, indépendamment l'un de l'autre, désignent hydrogène, un groupement alkyle inférieur en C₁-C₆, un groupement hydroxyle, alcoxy en C₁-C₆; au un atome d'halogène
R désigne un radical alkyle, linéaire ou ramifié en C₁-C₆, substitué par un ou plusieurs atomes d'halogène;
ainsi que leurs sels d'addition d'acides cosmétiquement ou pharmaceutiquement acceptables.

Les composés particulièrement préférés sont ceux dans lesquels les atomes d'halogène désignent fluor ou chlore; le groupement alkyle en C₁-C₆ représenté par R, désigne notamment un groupement mono- ou polyhalométhyle, -éthyle ou -propyle; R₁ et R₂ désignant de préférence hydrogène.

Les groupements R plus particulièrement préférés sont choisis parmi les groupements -CH₂CF₃, -CH₂-CF₂-CHF₂.

Les composés plus particulièrement préférés de l'invention sont constitués par le diamino-2,4(trifluoro-2 éthyloxy)-6 pyrimidine oxyde-3 et le diamino-2,4(tétrafluoro-2,2,3,3 propyloxy)-6 pyrimidine oxyde-3.

Les composés conformes à l'invention peuvent également exister sous la forme tautomère, répondant aux formules (IA) et (IB) suivantes : 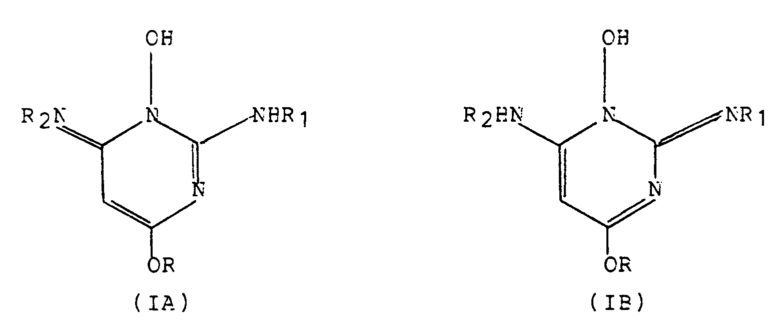
Ces formes tautomères (I), (IA), et (IB) peuvent être présentes dans des proportions variables et l'une peut être prépondérante par rapport aux autres.

Les composés conformes à l'invention pour lesquels R₁ et R₂ désignent hydrogène sont préparés en partant du diamino-2,4(dichloro-2,4 phénoxy)-6 pyrimidine oxyde-3 ou du diamino-2,4 chloro-6 pyrimidine oxyde-3 que l'on fait réagir avec un alcoolate de formule RO^{⊖}Y^{⊕} où R a la signification indiquée ci-dessus et Y est un cation alcalin tel que le sodium, le potassium, le lithium.

Les composés pour lesquels R₁ et R₂ sont des groupements carbamoyle, alcoxycarbonyle ou acyle, sont obtenus à partir des dérivés diamino-2,4 haloalcoxy-6 pyrimidine oxyde-3 correspondants dont la préparation est décrite ci-après.

L'obtention de dérivés pour lesquels R₁ et R₂ sont des groupements carbamoyle, est généralement réalisée en faisant réagir un chlorure de carbamoyle sur le dérivé diamino-2,4 haloalcoxy-6 pyrimidine oxyde-3 correspondant dans un solvant polaire, tel que le diméthylsulfoxyde, à une température comprise entre 0 et 100°C et plus particulièrement entre 20 et 70°C.

L'obtention de dérivés pour lesquels R₁ et R₂ sont des groupements alcoxycarbonyle, est généralement réalisée par l'action d'un excès d'ester chloroformique sur le dérivé diamino-2,4 haloalcoxy-6 pyrimidine oxyde-3 correspondant en opérant dans un solvant polaire aprotique, tel que le dichlorométhane en présence d'une amine tertiaire, telle que la triéthylamine ou la pyridine, à une température comprise entre 0 et 50°C.

L'obtention de dérivés pour lesquels R₁ et R₂ sont des groupements acyle, est généralement réalisée en faisant réagir un chlorure d'acide ou un anhydride sur le dérivé diamino-2,4 haloalcoxy-6 pyrimidine oxyde-3 correspondant dans un solvant polaire aprotique comme le dichlorométhane, en présence d'une amine tertiaire telle que la triéthylamine ou la pyridine, à une température comprise entre 0 et 50°C.

A partir des composés de formule (I), on peut préparer leurs sels d'addition d'acides cosmétiquement ou pharmaceutiquement acceptables, tels que les sels des acides sulfurique, chlorhydrique, bromhydrique, phosphorique, acétique, benzoïque, salicylique, glycolique, succinique, nicotinique, tartrique, maléïque, pamoïque, méthane sulfonique, picrique, lactique, et.

Les composés conformes à l'invention peuvent être utilisés dans le domaine cosmétique ou pharmaceutique, notamment dans les applications topiques, et plus particulièrement dans le traitement ou la prévention de la chute des cheveux, et plus particulièrement de la pelade, de l'alopécie, ainsi que des dermatites desquamantes.

Ces compositions sont essentiellement caractérisées par le fait qu'elles contiennent dans un milieu physiologiquement acceptable, approprié pour une application topique, au moins un composé répondant à la formule (I) ou un de ses sels.

Ces compositions peuvent comporter à titre de milieu physiologiquement acceptable, tout milieu approprié pour l'application topique, soit en cosmétique, soit en pharmacie, et qui soit compatible avec la substance active.

Les composés conformes à l'invention peuvent se trouver dans ce milieu, soit à l'état dissous, soit à l'état dispersé, notamment sous forme micronisée.

Les compositions destinées à être utilisées en pharmacie se présentent sous forme d'onguent, de teinture, de crème, de pommade, de poudre, de timbre, de tampon imbibé, de solution, d'émulsion ou d'émulsion vésiculaire, de lotion, de gel, de spray ou de suspension. Elles peuvent être, soit anhydres, soit aqueuses, selon l'indication clinique.

Les composés sont présents dans ces compositions pharmaceutiques à des concentrations comprises entre 0,1 et 20% en poids, et en particulier comprises entre 0,2 et 10% en poids.

Les compositions cosmétiques sont notamment destinées à être utilisées sous forme de lotion, de gel, de savon, de shampooing, d'aérosol ou de mousse et contiennent, dans un support physiologiquement acceptable, au moins un composé de formule (I) ou l'un de ses sels.

La concentration des composés de formule (I) dans ces compositions est, de préférence, comprise entre 0,01 et 15% en poids et en particulier entre 0,05 et 10% en poids.

Les compositions conformes à l'invention peuvent contenir différents additifs habituellement utilisés en cosmétique ou en pharmacie et en particulier des substances actives, telles que des agents hydratants comme la thiamorpholine et ses dérivés ou l'urée; des agents antiséborrhéiques tels que la S-carboxyméthylcystéine, la S-benzylcystéamine et leurs dérivés; la thioxolone.

Les composés conformes à l'invention peuvent être associés à des composés améliorant encore leur activité sur la repousse et/ou sur le freinage de la chute des cheveux, tels que plus particulièrement les composés suivants :
- les esters d'acide nicotinique, dont plus particulièrement les nicotinates d'alkyle en C₁-C₆ et notamment le nicotinate de méthyle;
- les agents anti-inflammatoires stéroïdiens et non stèroïdiens bien connus dans l'état de la technique et en particulier l'hydrocortisone, ses sels et ses dérivés, l'acide niflumique, etc;
- les rétinoïdes et plus particulièrement l'acide t-trans rétinoïque appelé encore trétinoïne, l'isotrétinoïne, le rétinol ou vitamine A et ses dérivés, tels que l'acétate, le palmitate ou le propionate, le motrétinide, l'étrétinate, le t-trans rétinoate de zinc;
- les agents antibactériens choisis plus particulièrement parmi les macrolides, les pyranosides et les tétracyclines et notamment l'érythromycine;
- les agents antagonistes de calcium, tels que plus particulièrement la cinnarizine et le diltiazem;
- des hormones, telles que l'estriol ou des analogues ou la thyroxine et ses sels;
- des agents antiandrogènes, tels que l'oxendolone, la spironolactone, le diéthylstilbestrol;
- des capteurs de radicaux OH, tels que le diméthylsulfoxyde.

On peut également associer avec les composés de l'invention, éventuellement en mélange avec les autres, des composés tels que le diazoxyde correspondant au méthyl-3 chloro-7 2H benzothiadiazine 1,2,4-dioxyde-1,1; la spiroxasone ou 7-(acéthylthio)-4′,5′-dihydrospiro [androst 4-ène-17,2′-(3′H)furan]-3 one; des phospholipides, tels que la lécithine; les acides linoléique et linolénique; l'acide salicylique et ses dérivés décrits dans le brevet français 2 581 542, et plus particulièrement les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters, des lactones et leurs sels correspondants; l'anthraline ou le trihydroxy-1,8,9 anthracène, les caroténoïdes, acides eicosatétraynoïque et eicosatriynoïque, leurs esters et amides.

Les composés conformes à l'invention peuvent également être associés à des agents tensio-actifs dont plus particulièrement ceux choisis parmi les agents tensio-actifs non ioniques et amphotères.

Parmi les tensio-actifs non ioniques, on citera notamment les polyhydroxypropyléthers décrits dans les brevets français n° 1 477 048; 2 091 516; 2 169 787; 2 328 763; 2 574 786; les alkyl(C₈-C₉) phénols oxyéthylénés comportant de 1 à 100 moles d'oxyde d'éthylène et de préférence 5 à 35 moles d'oxyde d'éthylène; les alkylpolyglycosides de formule :

CₙH₂ₙ₊₁(C₆H₁₀O₅)ₓH (A)

dans laquelle n varie de 8 à 15 inclus et x de 1 à 10 inclus.

Parmi les agents tensio-actifs amphotères, on citera plus particulièrement les amphocarboxyglycinates et les amphocarboxypropionates définis dans le dictionnaire CTFA, 3ème édition, 1982, et vendus, notamment, sous la dénomination MIRANOL® par la Société MIRANOL.

Les composés, selon l'invention, peuvent être introduits dans des supports qui améliorent encore l'activité au niveau de la repousse, en présentant à la fois des propriétés avantageuses sur le plan cosmétique, telles que des mélanges volatils ternaires d'alkyléther d'alkylèneglycol ou de dialkylèneglycol (alkyle et alkylène de préférence en C₁ à C₄), d'alcool éthylique et d'eau, le solvant glycolique désignant plus particulièrement le monoéthyléther de l'éthylène glycol, le monométhyléther du propylèneglycol, le monoéthylether du diéthylèneglycol.

Les composés conformes à l'invention peuvent également être introduits dans des supports gélifiés ou épaissis, tels que des supports essentiellement aqueux gélifiés par des hétérobiopolysaccharides, tels que la gomme de xanthane ou les dérivés de cellulose, des supports hydroalcooliques gélifiés par des polyhydroxy éthylacrylate ou méthacrylate ou des supports essentiellement aqueux épaissis, en particulier par des acides polyacryliques réticulés par un agent polyfonctionnel, tel que les Carbopol vendus par la Société GOODRICH.

Ces compositions peuvent également contenir des agents conservateurs, des agents stabilisants, des agents régulateurs de pH, des agents modificateurs de pression osmotique, des agents émulsifiants, des filtres UVA et UVE, des agents antioxydants tels que l' α-tocophérol, le butylhydroxyanisole, le butylhydroxytoluène.

Le milieu physiologiquement acceptable peut être constitué par de l'eau ou un mélange d'eau et d'un solvant ou un mélange de solvants, les solvants étant choisis parmi les solvants organiques acceptables sur le plan cosmétique ou pharmaceutique et choisis plus particulièrement parmi les alcools inférieurs en C₁-C₄, comme l'alcool éthylique, l'alcool isopropylique, l'alcool tertiobutylique, les alkylèneglycols, les alkyléthers d'alkylèneglycol et de dialkylèneglycol, tels que le monoéthyléther d'éthylèneglycol, le monométhyléther de propylèneglycol, le monoéthyléther de diéthylèneglycol. Les solvants, lorsqu'ils sont présents, le sont dans des proportions comprises entre 1 et 80% en poids par rapport au poids total de la composition.

Les milieux physiologiquement acceptables peuvent être épaissis à l'aide d'agents épaississants habituellement utilisés en cosmétique ou pharmacie, et on peut plus particulièrement citer les hétérobiopolysaccharides tels que la gomme de xanthane, les scléroglucanes, les dérivés de cellulose comme les éthers de cellulose, les polymères acryliques, réticulés ou non.

Les épaississants sont présents de préférence dans des proportions comprises entre 0,1 et 5% en poids et en particulier entre 0,4 et 3% en poids par rapport au poids total de la composition.

L'invention a également pour objet un procédé de traitement cosmétique des cheveux ou du cuir chevelu, consistant à leur appliquer au moins une composition telle que définie ci-dessus, en vue d'améliorer l'esthétique de la chevelure.

Un autre objet de l'invention est constitué par l'utilisation de la composition définie ci-dessus, pour la préparation d'un médicament ayant pour effet d'induire ou de stimuler la croissance des cheveux et de freiner leur chute.

Le traitement consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant une durée de 1 à 6 mois.

Les compositions peuvent notamment être utilisées dans le traitement de la pelade, de la chute des cheveux, de la dermatite desquamante.

Les exemples suivants sont destinés à illustrer l'invention.

### EXEMPLE DE PREPARATION 1

### Diamino-2,4(tétrafluoro-2,2,3,3 propyloxy)-6 pyrimidine oxyde-3.

2 g de sodium sont introduits dans 100 g de tétrafluoro-2,2,3,3 propanol à 40°C. Après addition de 10 g de diamino-2,4 chloro-6 pyrimidine oxyde-3, le mélange réactionnel est maintenu 32 heures à 90°C.

Le milieu réactionnel est refroidi à 0°C puis filtré afin d'éliminer un insoluble. Le résidu d'évaporation à sec du filtrat est repris par 100 cm³ d'hexane. On élimine un insoluble par filtration. Le produit attendu est obtenu après évaporation à sec du filtrat. Après recristallisation, on obtient 11 g d'une poudre blanche, fondant à 148°C.

| Analyse élémentaire pour C₇H₈F₄N₄O₂ | | | | |
|---|---|---|---|---|
| | C | H | N | F |
| Calculé | 32,82 | 3,15 | 21,87 | 29,67 |
| Trouvé | 32,65 | 3,24 | 21,80 | 29,60 |

Le spectre ¹H RMN est conforme à la structure attendue.

### EXEMPLE DE PREPARATION 2

### Diamino-2,4(trifluoro-2 éthyloxy)-6 pyrimidine oxyde-3.

3 g de sodium sont introduits dans 50 ml de trifluoroéthanol à 40°C. Après addition de 10 g de diamino-2,4 chloro-6 pyrimidine oxyde-3, le mélange réactionnel est maintenu 20 heures à 85°C.

Le milieu réactionnel est refroidi à 0°C puis filtré afin d'éliminer un insoluble. Le résidu d'évaporation à sec du filtrat est repris par du dichlorométhane puis par de l'hexane afin d'éliminer le trifluoroéthanol n'ayant pas réagi. Le produit attendu sous forme de poudre blanche est recristallisé à chaud de l'acétonitrile. On obtient 4,2 g. Il fond à 205°C.

| Analyse élémentaire pour C₆H₇F₃N₄O₂ | | | | |
|---|---|---|---|---|
| | C | H | N | F |
| Calculé | 32,15 | 3,15 | 25,00 | 25,43 |
| Trouvé | 31,63 | 2,97 | 24,67 | 25,08 |

Le spectre ¹H RMN est conforme à la structure attendue.

### EXEMPLE DE PREPARATION 3

### Diacetamido-2,4 (trifluoro-2′,2′,2′éthoxy)-6 pyrimidine N-oxyde-3.

Dans un tricol de 250 ml saturé en atmosphère d'argon, sont placés 90 ml de dichlorométhane, 11,1 g de triéthylamine et 6 g de diamino-2,4(trifluoro-2′,2′,2′éthoxy)-6 pyrimidine N-oxyde-3. On additionne goutte à goutte au mélange refroidi entre 0 et 5°C, 8,65 g de chlorure d'acétyle.

Après 8 heures 30 minutes de réaction, on lave le milieu réactionnel par :
- 2 x 40 ml d'eau
- 60 ml d'une solution Na₂CO₃ à 1%
- 2 x 40 ml d'eau.

On sèche la phase organique sur Na₂SO₄. Après filtration du Na₂SO₄, le filtrat est concentré au tiers de solvant. On précipite le produit par addition de 250 ml d'hexane. Ce dernier est recristallisé dans un mélange hexane-acétone 1/2.

On obtient 2,25 g de diacetamido-2,4(trifluoro-2′,2′,2′éthoxy)-6 pyrimidine N-oxyde-3.
Rendement = 27%.
P.F. = 183-185°C.

| Analyse élémentaire pour C₁₀H₁₁F₃N₄O₄ ; M=308,2 g. | | | | |
|---|---|---|---|---|
| | C | H | N | F |
| Calculé | 38,97 | 3,60 | 18,18 | 18,49 |
| Trouvé | 39,55 | 3,66 | 18,11 | 18,05 |

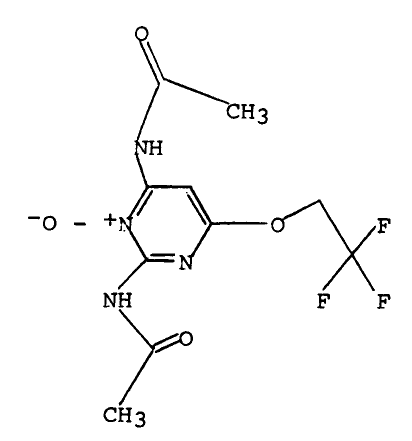

Les spectres de masse ¹H RMN sont conformes à la structure attendue.

### EXEMPLE DE PREPARATION 4

### Diméthoxycarbonylamino-2,4(trifluoro-2′,2′,2′éthoxy)-6 pyrimidine N-oxyde-3.

Dans un tricol de 250 ml saturé en atmosphère d'argon, sont placés 60 ml de dichlorométhane, 16,7 g de triéthylamine et 6 g de diamino-2,4(trifluoro-2′,2′,2′éthoxy)-6 pyrimidine N-oxyde-3. On additionne à ce mélange 15,6 g de chloroformiate de méthyle goutte à goutte entre 0 et 5°C.

Après 16 heures d'agitation à température ambiante, le milieu réactionnel est lavé avec :
- 4 x 20 ml d'une solution HCl à 1%
- 3 x 20 ml d'eau (jusqu'à neutralité de la phase aqueuse).

La phase organique est séchée sur Na₂SO₄ et le solvant est évaporé sous vide. Le brut recueilli est repris dans 50 ml d'éther éthylique et filtré. Après évaporation du filtrat, on obtient un liquide visqueux qui est porté au reflux dans 80 ml de méthanol. Il se forme un précipité que l'on filtre à chaud et que l'on sèche sous vide et sur P₂O₅ pendant 12 heures.

On recueille ainsi 5,4 g de diméthoxycarbonyl amino-2,4(trifluoro-2′,2′,2′éthoxy)-6 pyrimidine N-oxyde-3.
Rendement = 59%.
P.F. = 184-185°C.

| Analyse élémentaire pour C₁₀H₁₁F₃N₄O₆ ; M=340,2 g. | | | | |
|---|---|---|---|---|
| | C | H | N | F |
| Calculé | 35,30 | 3,26 | 16,47 | 16,75 |
| Trouvé | 35,36 | 3,21 | 16,27 | 16,78 |

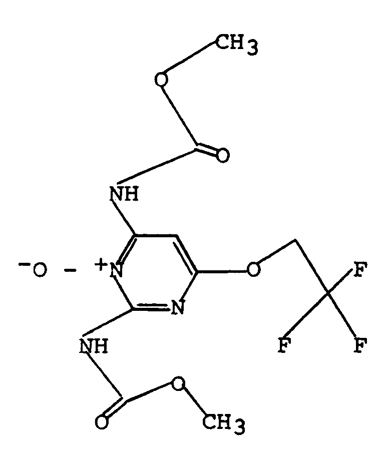

Les spectres de masse ¹H RMN sont conformes à la structure attendue.

### EXEMPLE DE COMPOSITION 1

On prépare la composition suivante :

| | |
|---|---|
| - Diamino-2,4(tétrafluoro-2,2,3,3 propyloxy)-6 pyrimidine oxyde-3 | 6,0 g |
| - Mélange éthanol absolu/propylène glycol (95/5) | qsp 100,0 g |

### EXEMPLE DE COMPOSITION 2

On prépare la composition suivante :

| | |
|---|---|
| - Diamino-2,4(trifluoro-2 éthyloxy)-6 pyrimidine oxyde-3 | 10,0 g |
| - Propylèneglycol | 20,0 g |
| - Ethanol | 50,0 g |
| - Eau | qsp 100,0 g |

Ces deux compositions se présentent sous forme de lotion.

1 à 2 g de cette composition sont appliqués sur les zones alopéciques du cuir chevelu, éventuellement accompagné d'un massage pour favoriser la pénétration, à raison de une à deux applications par jour, pendant 3 mois de traitement.

On prépare de la même façon des compositions sous forme de lotion conformes à l'exemple 1, en remplaçant le dérivé de pyrimidine oxyde-3 par respectivement les composés des exemples de préparation 3 et 4.

## Revendications (Revendications pour l'(les) Etat(s) contractant(s) suivant(s): DE, GB, FR, IT, NL, SE, CH, BE, AT, DK)

1. Composé caractérisé par le fait qu'il répond à la formule : 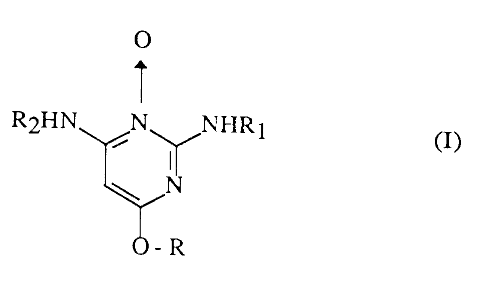 dans laquelle :
R₁ et R₂ désignent, indépendamment l'un de l'autre, un atome d'hydrogène ou bien un groupement carbamoyle de formule : 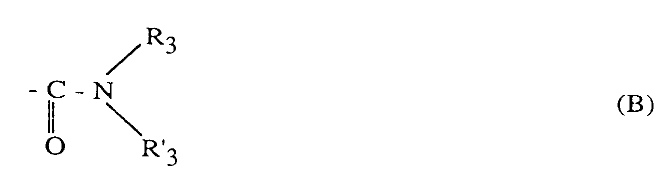 avec R'₃ désignant hydrogène ou R₃;
un groupement alcoxycarbonyle de formule : 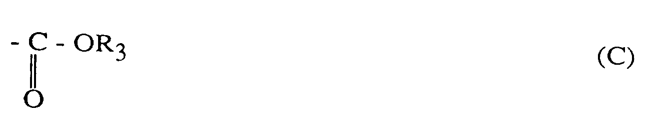 ou un groupement acyle de formule : 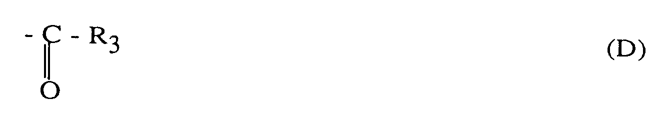 dans lesquels R₃ désigne un radical alkyle linéaire ou ramifié en C₁-C₁₈, un groupement alcényle en C₂-C₁₈, un groupement cycloalkyle en C₅-C₈; R₃ peut également désigner un radical aryle ou aralkyle répondant à la formule : 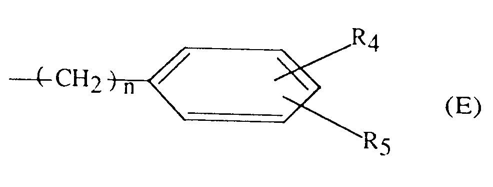 dans laquelle :
n est compris entre 0 et 4;
R₄ et/ou R₅, indépendamment l'un de l'autre, désignent hydrogène, un groupement alkyle inférieur en C₁-C₆, un groupement hydroxyle, alcoxy en C₁-C₆ ou un atome d'halogène,
R désigne un radical alkyle, linéaire ou ramifié en C₁-C₆, substitué par un ou plusieurs atomes d'halogène ainsi que leurs sels d'addition d'acides.

2. Composé selon la revendication 1, caractérisé par le fait que le radical R est un groupement mono- ou polyhalométhyle, -éthyle ou propyle, et le ou les atomes d'halogène sont choisis parmi le fluor et le chlore.

3. Composé selon la revendication 1 ou 2, caractérisé par le fait qu'il s'agit du diamino-2,4 (trifluoro-2 éthyloxy)-6 pyrimidine oxyde-3, du diamino-2,4(tétrafluoro-2,2,3,3 propyloxy)-6 pyrimidine oxyde-3.

4. Composition destinée à être utilisée en application topique, caractérisée par le fait qu'elle contient, dans un milieu physiologiquement acceptable, au moins un composé de formule (I) tel que défini dans l'une quelconque des revendications 1 à 3.

5. Composition selon la revendication 4, caractérisée par le fait qu'elle se présente sous forme d'onguent, de teinture, de crème, de pommade, de poudre, de timbre, de tampon imbibé, de solution, d'émulsion, d'émulsion vésiculaire, de lotion, de gel, de spray ou de suspension anhydre ou aqueuse, en vue de son application pharmaceutique et qu'elle contient au moins un composé de formule (I) tel que défini dans l'une quelconque des revendications 1 à 3.

6. Composition selon la revendication 4 ou 5, caractérisée par le fait que les composés de formule (I) sont présents à des concentrations comprises entre 0,1 et 20% en poids par rapport au poids total de la composition et en particulier entre 0,2 et 10% en poids.

7. Composition destinée à être utilisée en cosmétique, telle que définie dans la revendication 4, caractérisée par le fait qu'elle se présente sous forme de lotion, de gel, de savon, de shampooing, d'aérosol ou de mousse et qu'elle contient, dans un support acceptable sur le plan cosmétique, au moins un composé tel que défini dans l'une quelconque des revendications 1 à 3, à une concentration comprise entre 0,01 et 15% en poids.

8. Composition selon l'une quelconque des revendications 4 à 7, caractérisée par le fait qu'elle contient, en plus des agents hydratants, des agents antiséborrhéiques.

9. Composition selon l'une quelconque des revendications 4 à 8, caractérisée par le fait qu'elle contient en plus des esters d'acide nicotinique, des agents anti-inflammatoires stéroïdiens ou non-stéroïdiens, des rétinoïdes, des agents antibactériens, des agents antagonistes du calcium, des hormones, des agents antiandrogènes, des capteurs de radicaux OH.

10. Composition selon l'une quelconque des revendications 4 à 8, caractérisée par le fait qu'elle contient en plus des composés choisis parmi le diazoxyde, la spiroxasone, des phospholipides, des acides linolénique et linoléique, l'acide salicylique et ses dérivés, des acides hydroxycarboxyliques ou cétocarboxyliques, leurs esters, des lactones et leurs sels correspondants, l'anthraline ou le trihydroxy-1,8,9 anthracène, les caroténoïdes, les acides eicosatétraynoïques-5,8,11,14 eicosatriynoïques-5,8,11, leurs esters et amides.

11. Composition selon l'une quelconque des revendications 4 à 10, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

12. Composition selon la revendication 11, caractérisée par le fait que les solvants sont choisis parmi les alcools inférieurs en C₁-C₄, les alkylèneglycols, les alkyléthers de mono- et de dialkylèneglycol.

13. Composition selon l'une quelconque des revendications 4 à 12, caractérisée par le fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants, des filtres UV-A et UV-B, des agents anti-oxydants.

14. Composition selon l'une quelconque des revendications 4 à 13, caractérisée par le fait qu'elle contient également des agents tensio-actifs choisis parmi les agents tensio-actifs non-ioniques et amphotères.

15. Composition selon l'une quelconque des revendications 4 à 14, pour son application comme médicament destiné à être utilisé dans le traitement thérapeutique de la chute des cheveux.

16. Procédé de traitement cosmétique des cheveux ou du cuir chevelu, caractérisé par le fait que l'on applique la composition telle que définie dans l'une quelconque des revendications 4 à 14.

17. Utilisation de la composition telle que définie dans l'une quelconque des revendications 4 à 14, pour la préparation d'un médicament destiné à être utilisé dans le traitement thérapeutique de la chute des cheveux.

## Revendications (Revendications pour l'(les) Etat(s) contractant(s) suivant(s): ES)

1. Procédé de préparation de composés de formule : 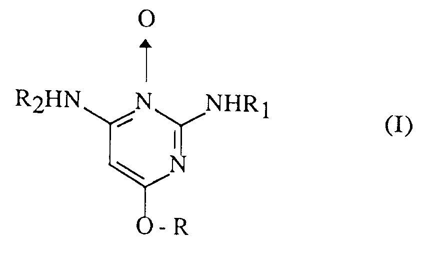 dans laquelle :
R₁ et R₂ désignent, indépendamment l'un de l'autre, un atome d'hydrogène ou bien un groupement carbamoyle de formule : 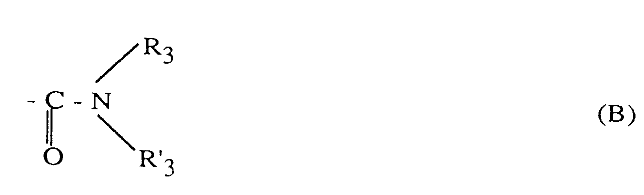 avec R'₃ désignant hydrogène ou R₃;
un groupement alcoxycarbonyle de formule : 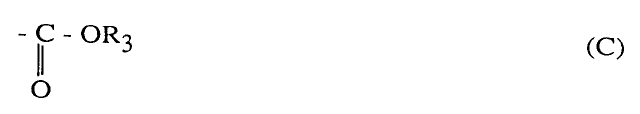 ou un groupement acyle de formule : 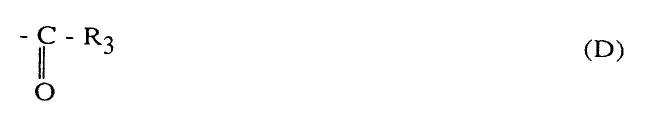 dans lesquels R₃ désigne un radical alkyle linéaire ou ramifié en C₁-C₁₈, un groupement alcényle en C₂-C₁₈, un groupement cycloalkyle en C₅-C₈; R₃ peut également désigner un radical aryle ou aralkyle répondant à la formule : 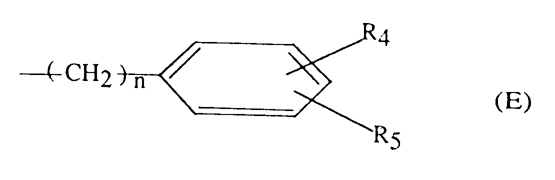 dans laquelle :
n est compris entre 0 et 4;
R₄ et/ou R₅, indépendamment l'un de l'autre, désignent hydrogène, un groupement alkyle inférieur en C₁-C₆, un groupement hydroxyle, alcoxy en C₁-C₆ ou un atome d'halogène,
R désigne un radical alkyle, linéaire ou ramifié en C₁-C₆, substitué par un ou plusieurs atomes d'halogène, ainsi que leurs sels d'addition d'acides physiologiquement acceptables,
caractérisé par le fait que :
a) pour obtenir les composés de formule (I) pour lesquels R₁ et R₂ désignent hydrogène, on fait réagir le diamino-2,4(dichloro-2,4 phénoxy)-6 pyrimidine oxyde-3 ou le diamino 2,4-chloro-6 pyrimidine oxyde-3 avec un alcoolate RO ⁻ Y ⁺ où R a la signification indiquée ci-dessus et Y est un cation alcalin;
b) pour obtenir un composé de formule (I) où R₁ et R₂ désignent carbamoyle, on fait réagir le dérivé diamino-2,4 haloalcoxy-6 pyrimidine oxyde-3 correspondant avec un chlorure de carbamoyle dans un solvant polaire à une température comprise entre 0 et 100°C;
c) pour obtenir un composé de formule (I) où R₁ et R₂ désignent des groupements alcoxycarbonyle, on fait réagir le dérivé diamino-2,4 haloalcoxy-6 pyrimidine oxyde-3 correspondant avec un excès d'ester chloroformique dans un solvant polaire aprotique à une température entre 0 et 50°C;
d) pour obtenir un composé de formule (I) où R₁ et R₂ désignent des groupements acyle, on fait réagir le dérivé diamino-2,4 haloalcoxy-6 pyrimidine oxyde-3 correspondant avec un chlorure d'acide ou un anhydride dans un solvant polaire aprotique à une température comprise entre 0 et 50°C.

2. Composition destinée à être utilisée en application topique, caractérisée par le fait qu'elle contient, dans un milieu physiologiquement acceptable, au moins un composé de formule (I) tel que défini dans la revendication 1.

3. Composition selon la revendication 2, caractérisée par le fait qu'elle se présente sous forme d'onguent, de teinture, de crème, de pommade, de poudre, de timbre, de tampon imbibé, de solution, d'émulsion, d'émulsion vésiculaire, de lotion, de gel, de spray ou de suspension anhydre ou aqueuse, et qu'elle contient au moins un composé de formule (I) tel que défini dans la revendication 1.

4. Composition selon la revendication 2 ou 3, caractérisée par le fait que les composés de formule (I) sont présents à des concentrations comprises entre 0,1 et 20% en poids par rapport au poids total de la composition.

5. Composition telle que définie dans la revendication 2, caractérisée par le fait qu'elle se présente sous forme de lotion, de gel, de savon, de shampooing, d'aérosol ou de mousse et qu'elle contient, dans un support acceptable sur le plan cosmétique, au moins un composé tel que défini dans la revendication 1, à une concentration comprise entre 0,01 et 15% en poids.

6. Composition selon l'une quelconque des revendications 2 à 5, caractérisée par le fait qu'elle contient, en plus des agents hydratants, des agents antiséborrhéiques.

7. Composition selon l'une quelconque des revendications 2 à 6, caractérisée par le fait qu'elle contient en plus des esters d'acide nicotinique, des agents anti-inflammatoires stéroïdiens ou non-stéroïdiens, des rétinoïdes, des agents antibactériens, des agents antagonistes du calcium, des hormones, des agents antiandrogènes, des capteurs de radicaux OH.

8. Composition selon l'une quelconque des revendications 2 à 7, caractérisée par le fait qu'elle contient en plus des composés choisis parmi le diazoxyde, la spiroxasone, des phospholipides, des acides linolénique et linoléique, l'acide salicylique et ses dérivés, des acides hydroxycarboxyliques ou cétocarboxyliques, leurs esters, des lactones et leurs sels correspondants, l'anthraline ou le trihydroxy-1,8,9 anthracène, les caroténoïdes, les acides eicosatétraynoïques-5,8,11,14 eicosatriynoïques-5,8,11, leurs esters et amides.

9. Composition selon l'une quelconque des revendications 4 à 8, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

10. Composition selon la revendication 9, caractérisée par le fait que les solvants sont choisis parmi les alcools inférieurs en C₁-C₄, les alkylèneglycols, les alkyléthers de mono- et de dialkylèneglycol.

11. Composition selon l'une quelconque des revendications 4 à 10, caractérisée par le fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants, des filtres UVA et UVB, des agents anti-oxydants.

12. Composition selon l'une quelconque des revendications 4 à 11, caractérisée par le fait qu'elle contient également des agents tensio-actifs choisis parmi les agents tensio-actifs non-ioniques et amphotères.

13. Procédé de traitement cosmétique des cheveux ou du cuir chevelu, caractérisé par le fait que l'on applique la composition telle que définie dans l'une quelconque des revendications 4 à 12.

14. Utilisation de la composition telle que définie dans l'une quelconque des revendications 4 à 12, pour la préparation d'un médicament destiné à être utilisé dans le traitement thérapeutique de la chute des cheveux.

15. Procédé de préparation d'une composition destinée à être appliquée par voie topique, caractérisé par le fait que l'on incorpore dans un milieu physiologiquement acceptable, au moins un composé de formule (I) tel que défini dans la revendication 1.

## Claims (Claims for the following Contracting State(s): DE, GB, FR, IT, NL, SE, CH, BE, AT, DK)

1. Compound, characterized in that it corresponds to the formula: 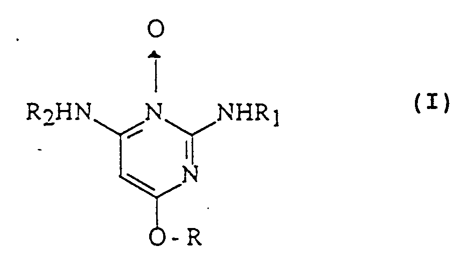 in which:
R₁ and R₂ denote, independently of one another, a hydrogen atom or alternatively a carbamoyl group of formula: 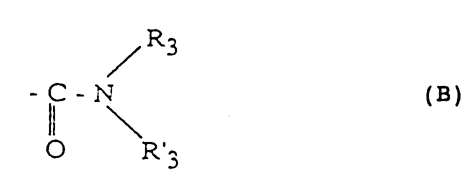 with R'₃ denoting hydrogen or R₃;
an alkoxycarbonyl group of formula: 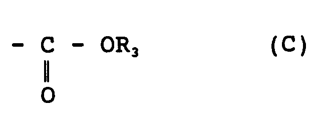 or an acyl group of formula: 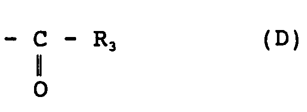 in which R₃ denotes a linear or branched C₁-C₁₈ alkyl radical, a C₂-C₁₈ alkenyl group or a C₅-C₈ cycloalkyl group; R₃ can also denote an aryl or aralkyl radical corresponding to the formula: 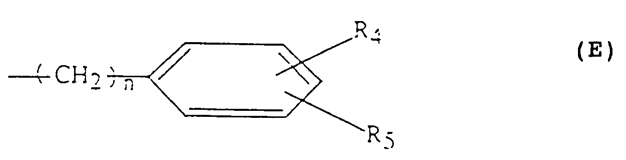 in which:
n is between 0 and 4;
R₄ and/or R₅, independently of one another, denote hydrogen, a C₁-C₆ lower alkyl group, a hydroxyl or C₁-C₆ alkoxy group or a halogen atom, and
R denotes a linear or branched C₁-C₆ alkyl radical substituted with one or more halogen atoms as well as their acid addition salts.

2. Compound according to Claim 1, characterized in that the radical R is a mono- or polyhalomethyl, -ethyl or -propyl group, and the halogen atom or atoms are selected from fluorine and chlorine.

3. Compound according to Claim 1 or 2, characterized in that it is 2,4-diamino-6-(2-trifluoroethyloxy)pyrimidine 3-oxide or 2,4-diamino-6-(2,2,3,3-tetrafluoropropyloxy)pyrimidine 3-oxide.

4. Composition intended for use in topical application, characterized in that it contains at least one compound of formula (I) as defined in any one of Claims 1 to 3 in a physiologically acceptable medium.

5. Composition according to Claim 4, characterized in that it is presented in the form of an ointment, tincture, cream, pomade, powder, patch, impregnated pad, solution, emulsion, vesicular emulsion, lotion, gel, spray or anhydrous or aqueous suspension, for the purpose of its pharmaceutical application, and in that it contains at least one compound of formula (I) as defined in any one of Claims 1 to 3.

6. Composition according to Claim 4 or 5, characterized in that the compounds of formula (I) are present at concentrations of between 0.1 and 20% by weight relative to the total weight of the composition, and especially between 0.2 and 10% by weight.

7. Composition intended for use in cosmetics, as defined in Claim 4, characterized in that it is presented in the form of a lotion, gel, soap, shampoo, aerosol or mousse, and in that it contains at least one compound as defined in any one of Claims 1 to 3, at a concentration of between 0.01 and 15% by weight, in a vehicle acceptable from the cosmetic standpoint.

8. Composition according to any one of Claims 4 to 7, characterized in that it also contains hydrating agents, antiseborrhoeic agents.

9. Composition according to any one of Claims 4 to 8, characterized in that it also contains nicotinic acid esters, steroidal or non-steroidal anti-inflammatory agents, retinoids, antibacterial agents, calcium antagonists, hormones, anti-androgens, OH radical trapping agents.

10. Composition according to any one of Claims 4 to 8, characterized in that it also contains compounds selected from diazoxide, spiroxasone, phospholipids, linolenic and linoleic acids, salicylic acid and its derivatives, hydroxy-carboxylic or keto-carboxylic acids, their esters, lactones and their corresponding salts, anthralin or 1,8,9-trihydroxyanthracene, carotenoids, and 5,8,11,14-eicosatetraynoic and 5,8,11-eicosatriynoic acids, their esters and amides.

11. Composition according to any one of Claims 4 to 10, characterized in that the physiologically acceptable medium consists of water, a mixture of water and one or more organic solvent(s), or of a mixture of organic solvents, the organic solvents being pharmaceutically or cosmetically acceptable.

12. Composition according to Claim 11, characterized in that the solvents are selected from C₁-C₄ lower alcohols, alkylene glycols, and mono- and dialkylene glycol alkyl ethers.

13. Composition according to any one of Claims 4 to 12, characterized in that the physiologically acceptable medium is thickened by means of thickening and/or gelling agents, and contains preservative agents, stabilizing agents, pH-regulating agents, osmotic pressure-modifying agents, emulsifying agents, UVA and UVB screening agents and antioxidant agents.

14. Composition according to any one of Claims 4 to 13, characterized in that it also contains surfactant agents selected from nonionic and amphoteric surfactant agents.

15. Composition according to any one of Claims 4 to 14, for its application as a medicinal product intended for use in the therapeutic treatment of hair loss.

16. Process for cosmetic treatment of the hair or scalp, characterized in that the composition as defined in any one of Claims 4 to 14 is applied.

17. Use of the composition as defined in any one of Claims 4 to 14, for the preparation of a medicinal product intended for use in the therapeutic treatment of hair loss.

## Claims (Claims for the following Contracting State(s): ES)

1. Process for the preparation of compounds of formula: 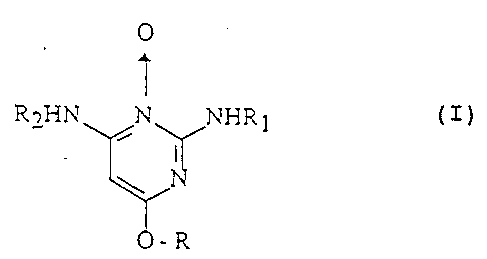 in which:
R₁ and R₂ denote, independently of one another, a hydrogen atom or alternatively a carbamoyl group of formula: 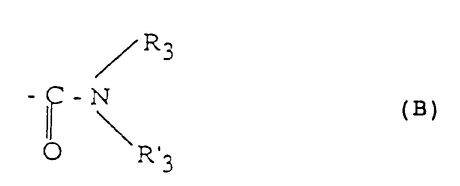 with R'₃ denoting hydrogen or R₃;
an alkoxycarbonyl group of formula: 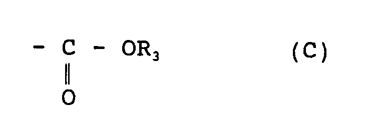 or an acyl group of formula: 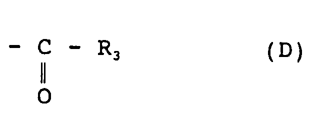 in which R₃ denotes a linear or branched C₁-C₁₈ alkyl radical, a C₂-C₁₈ alkenyl group or a C₅-C₈ cycloalkyl group; R₃ can also denote an aryl or aralkyl radical corresponding to the formula: 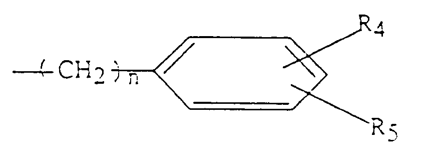 in which:
n is between 0 and 4;
R₄ and/or R₅, independently of one another, denote hydrogen, a C₁-C₆ lower alkyl group, a hydroxyl or C₁-C₆ alkoxy group or a halogen atom, and
R denotes a linear or branched C₁-C₆ alkyl radical substituted with one or more halogen atoms, as well as their addition salts with physiologically acceptable acids,
characterized in that:
a) in order to obtain the compounds of formula (I) for which R₁ and R₂ denote hydrogen, the 2,4-diamino-6-(2,4-dichlorophenoxy)pyrimidine 3-oxide or 2,4-diamino-6-chloropyrimidine 3-oxide is reacted with an alcoholate RO⁻ Y⁺ where R has the meaning indicated above and Y is an alkali metal cation;
b) in order to obtain a compound of formula (I) wherein R₁ and R₂ denote carbamoyl, the corresponding 2,4-diamino-6-haloalkoxypyrimidine 3-oxide derivative is reacted with a carbamoyl chloride in a polar solvent at a temperature of between 0 and 100°C;
c) in order to obtain a compound of formula (I) wherein R₁ and R₂ denote alkoxycarbonyl groups, the corresponding 2,4-diamino-6-haloalkoxypyrimidine 3-oxide derivative is reacted with an excess of chloroformic ester in an aprotic polar solvent at a temperature between 0 and 50°C;
d) in order to obtain a compound of formula (I) wherein R₁ and R₂ denote acyl groups, the corresponding 2,4-diamino-6-haloalkoxypyrimidine 3-oxide derivative is reacted with an acid chloride or an anhydride in an aprotic polar solvent at a temperature of between 0 and 50°C.

2. Composition intended for use in topical application, characterized in that it contains at least one compound of formula (I) as defined in Claim 1 in a physiologically acceptable medium.

3. Composition according to Claim 2, characterized in that it is presented in the form of an ointment, tincture, cream, pomade, powder, patch, impregnated pad, solution, emulsion, vesicular emulsion, lotion, gel, spray or anhydrous or aqueous suspension, and in that it contains at least one compound of formula (I) as defined in Claim 1.

4. Composition according to Claim 2 or 3, characterized in that the compounds of formula (I) are present at concentrations of between 0.1 and 20% by weight relative to the total weight of the composition.

5. Composition as defined in Claim 2, characterized in that it is presented in the form of a lotion, gel, soap, shampoo, aerosol or mousse, and in that it contains at least one compound as defined in Claim 1, at a concentration of between 0.01 and 15% by weight, in a vehicle acceptable from the cosmetic standpoint.

6. Composition according to any one of Claims 2 to 5, characterized in that it also contains hydrating agents, antiseborrhoeic agents.

7. Composition according to any one of Claims 2 to 6, characterized in that it also contains nicotinic acid esters, steroidal or non-steroidal anti-inflammatory agents, retinoids, antibacterial agents, calcium antagonists, hormones, anti-androgens, OH radical trapping agents.

8. Composition according to any one of Claims 2 to 7, characterized in that it also contains compounds selected from diazoxide, spiroxasone, phospholipids, linolenic and linoleic acids, salicylic acid and its derivatives, hydroxy-carboxylic or keto-carboxylic acids, their esters, lactones and their corresponding salts, anthralin or 1,8,9-trihydroxyanthracene, carotenoids, and 5,8,11,14-eicosatetraynoic and 5,8,11-eicosatriynoic acids, their esters and amides.

9. Composition according to any one of Claims 4 to 8, characterized in that the physiologically acceptable medium consists of water, a mixture of water and one or more organic solvent(s), or of a mixture of organic solvents, the organic solvents being pharmaceutically or cosmetically acceptable.

10. Composition according to Claim 9, characterized in that the solvents are selected from C₁-C₄ lower alcohols, alkylene glycols, and mono- and dialkylene glycol alkyl ethers.

11. Composition according to any one of Claims 4 to 10, characterized in that the physiologically acceptable medium is thickened by means of thickening and/or gelling agents, and contains preservative agents, stabilizing agents, pH-regulating agents, osmotic pressure-modifying agents, emulsifying agents, UVA and UVB screening agents and antioxidant agents.

12. Composition according to any one of Claims 4 to 11, characterized in that it also contains surfactant agents selected from nonionic and amphoteric surfactant agents.

13. Process for cosmetic treatment of the hair or scalp, characterized in that the composition as defined in any one of Claims 4 to 12 is applied.

14. Use of the composition as defined in any one of Claims 4 to 12, for the preparation of a medicinal product intended for use in the therapeutic treatment of hair loss.

15. Process for the preparation of a composition intended for topical application, characterized in that at least one compound of formula (I) as defined in Claim 1 is incorporated into a physiologically acceptable medium.

## Patentansprüche (Patentansprüche für folgende(n) Vertragsstaat(en): DE, GB, FR, IT, NL, SE, CH, BE, AT, DK)

1. Verbindung der Formel: 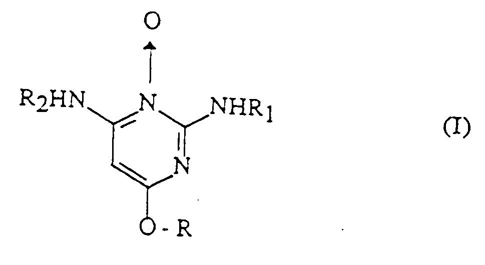 worin:
R₁ und R₂, unabhängig voneinander, ein Wasserstoffatom oder auch eine Carbamoylgruppe der Formel: 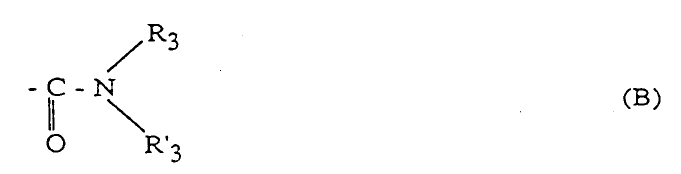 mit R'₃, das Wasserstoff oder R₃ bedeutet,
eine Alkoxycarbonylgruppe der Formel: 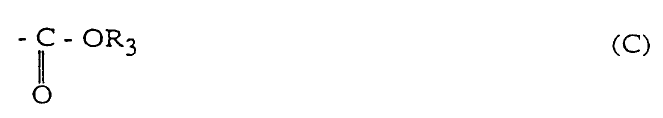 oder eine Acylgruppe der Formel: 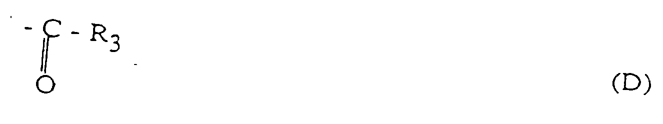 darstellen, in denen R₃ einen linearen oder verzweigten C₁₋₁₈-Alkylrest, eine C₂₋₁₈-Alkenylgruppe, eine C₅₋₈-Zykloalkylgruppe bedeutet, wobei R₃ auch einen Aryl- oder Aralkylrest der Formel: 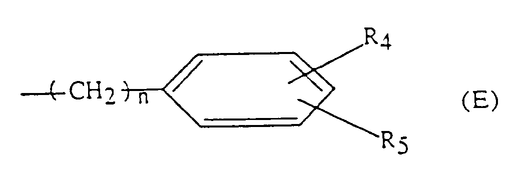 bedeuten kann, worin:
n 0 bis 4 beträgt und
R₄ und/oder R₅, unabhängig voneinander, Wasserstoff, eine C₁₋₆-Niedrigalkyl-, Hydroxyl-, C₁₋₆-Alkoxygruppe oder ein Halogenatom bedeuten,
und worin R einen linearen oder verzweigten C₁₋₆-Alkylrest darstellt, der mit einem oder mehreren Halogenatom(en) substituiert ist,
sowie deren Säureadditionssalze.

2. Verbindung gemäß Anspruch 1,
dadurch **gekennzeichnet,** daß
der Rest R eine Mono- oder Polyhalomethyl-, -ethyl- oder - propylgruppe ist und das oder die Halogenatom(e) aus Fluor und Chlor ausgewählt sind.

3. Verbindung gemäß Anspruch 1 oder 2,
dadurch **gekennzeichnet,** daß
es sich um 2,4-Diamino-6-(2',2',2'-trifluorethoxy)pyrimidin-3-N-oxid oder um 2,4-Diamino-6-(2',2',3',3'-tetrafluorpropoxy)pyrimidin-3-N-oxid handelt.

4. Zusammensetzung zur topischen Aufbringung,
dadurch **gekennzeichnet,** daß
sie in einem physiologisch verträglichen Milieu mindestens eine in jedem der Ansprüche 1 bis 3 definierte Verbindung der Formel (I) enthält.

5. Zusammensetzung gemäß Anspruch 4,
dadurch **gekennzeichnet,** daß
sie zu einer pharmazeutischen Anwendung in Form einer Salbe, Tinktur, Creme, Pommade, eines Pulvers, Tupfers, getränkten Tampons, einer Lösung, Emulsion, bläschenartigen Emulsion, Lotion, eines Gels, eines Spray oder einer wasserfreien oder wässrigen Suspension vorliegt und mindestens eine in jedem der Ansprüche 1 bis 3 definierte Verbindung der Formel (I) enthält.

6. Zusammensetzung gemäß Anspruch 4 oder 5,
dadurch **gekennzeichnet,** daß
die Verbindungen der Formel (I) in Konzentrationen von 0,1 bis 20 und insbesondere von 0,2 bis 10 Gew.%, bezogen auf Gesamtgewicht der Zusammensetzung, vorhanden sind.

7. Zusammensetzung gemäß Anspruch 4 zur Verwendung in der Kosmetik,
dadurch **gekennzeichnet,** daß
sie in Form einer Lotion, eines Gels, einer Seife, eines Shampoo, Aerosols oder Schaums vorliegt und, in einem auf dem kosmetischen Plan zulässigen Trägermedium, mindestens eine in jedem der Ansprüche 1 bis 3 definierte Verbindung in einer Konzentration von 0,01 bis 15 Gew.% enthält.

8. Zusammensetzung gemäß jedem der Ansprüche 4 bis 7,
dadurch **gekennzeichnet,** daß
sie, zusätzlich zu hydratisierenden Mitteln, antiseborrheische Mittel enthält.

9. Zusammensetzung gemäß jedem der Ansprüche 4 bis 8,
dadurch **gekennzeichnet,** daß
sie zusätzlich Nikotinsäureester, steroide oder nichtsteroide entzündungshemmende Mittel, Retinoide, antibakterielle Mittel, Calcium-Antagonisten, Hormone, antiandrogene Mittel, Abfangmittel von OH-Radikalen enthält.

10. Zusammensetzung gemäß jedem der Ansprüche 4 bis 8,
dadurch **gekennzeichnet,** daß
sie zusätzlich Verbindungen enthält, ausgewählt aus Diazoxid, Spiroxason, Phospholipiden, Linolen- und Linolsäuren, Salicylsäure und ihren Derivaten, Hydroxycarboxyl- oder Ketocarboxylsäuren, deren Estern, aus Lactonen und deren entsprechenden Salzen, Anthralin oder 1,8,9-Trihydroxyanthracen, Karotinoiden, Eicosa-5,8,11,14-tetrain-, Eicosa-5,8-11-triinsäuren, deren Estern und Amiden.

11. Zusammensetzung gemäß jedem der Ansprüche 4 bis 10,
dadurch **gekennzeichnet**, daß
das physiologisch zulässige Milieu aus Wasser, einer Mischung aus Wasser und einem oder mehreren organischen Lösungsmittel(n) oder aus einer Mischung organischer Lösungsmittel zusammengesetzt ist, wobei die organischen Lösungsmittel pharmazeutisch oder kosmetisch zulässig sind.

12. Zusammensetzung gemäß Anspruch 11,
dadurch **gekennzeichnet,** daß
die Lösungsmittel aus C₁₋₄-Niedrigalkoholen, Alkylenglycolen, Alkylethern vom Mono- und Dialkylenglycol ausgewählt sind.

13. Zusammensetzung gemäß jedem der Ansprüche 4 bis 12,
dadurch **gekennzeichnet,** daß
das physiologisch zulässige Milieu mit verdickenden und/oder gelierenden Mitteln verdickt ist und Konservierungs-, Stabilisierungs-, pH-Regulierungs-, Modifizierungsmittel des osmotischen Drucks, Emulgiermittel Filterstoffe für UV-A und UV-B, Antioxidantien enthält.

14. Zusammensetzung gemäß jedem der Ansprüche 4 bis 13,
dadurch **gekennzeichnet,** daß
sie auch oberflächenaktive Mittel enthält, die aus nichtionischen und amphoteren oberflächenaktiven Mitteln ausgewählt sind.

15. Zusammensetzung gemäß jedem der Ansprüche 4 bis 14 zur Anwendung als Medikament, das dazu bestimmt ist, in der therapeutischen Behandlung des Ausfalls der Haare verwendet zu werden.

16. Verfahren zur kosmetischen Behandlung der Haare oder der behaarten Haut,
dadurch **gekennzeichnet,** daß
man die in jedem der Ansprüche 4 bis 14 definierte Zusammensetzung zur Anwendung bringt.

17. Verwendung der gemäß jedem der Ansprüche 4 bis 14 definierten Zusammensetzung zur Herstellung eines Medikaments zur therapeutischen Behandlung des Ausfalls der Haare.

## Patentansprüche (Patentansprüche für folgende(n) Vertragsstaat(en): ES)

1. Verfahren zur Herstellung von Verbindungen der Formel: 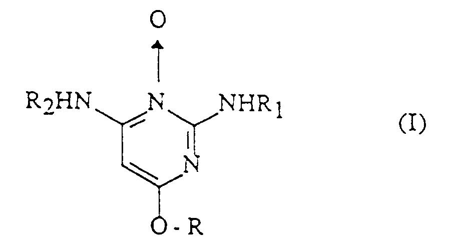 worin:
R₁ und R₂, unabhängig voneinander, ein Wasserstoffatom oder auch eine Carbamoylgruppe der Formel: 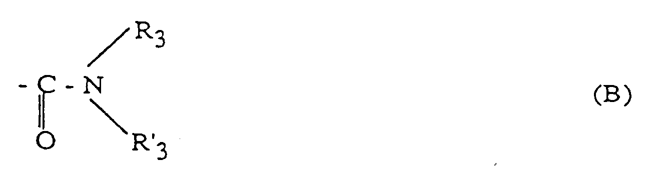 mit R'₃, das Wasserstoff oder R₃ bedeutet,
eine Alkoxycarbonylgruppe der Formel: 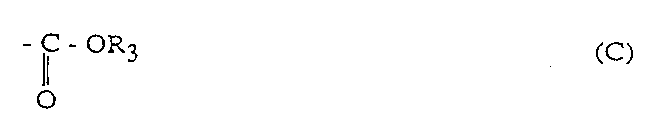 oder eine Acylgruppe der Formel: 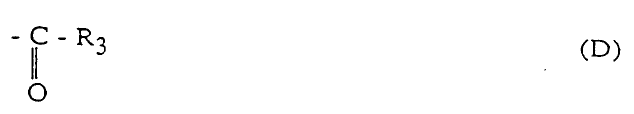 darstellen, in denen R₃ einen linearen oder verzweigten C₁₋₁₈-Alkylrest, eine C₂₋₁₈-Alkenylgruppe, eine C₅₋₈-Zykloalkylgruppe bedeutet, wobei R₃ auch einen Aryl- oder Aralkylrest der Formel: 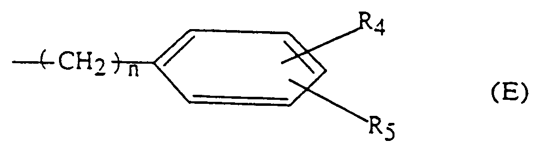 bedeuten kann, worin:
n 0 bis 4 beträgt und
R₄ und/oder R₅, unabhängig voneinander, Wasserstoff, eine C₁₋₆-Niedrigalkyl-, Hydroxyl-, C₁₋₆-Alkoxygruppe oder ein Halogenatom bedeuten,
und worin R einen linearen oder verzweigten C₁₋₆-Alkylrest darstellt, der mit einem oder mehreren Halogenatom(en) substituiert ist,
sowie von deren physiologisch zulässigen Säureadditionssalzen,
dadurch **gekennzeichnet**, daß man:
(a) zum Erhalt von Verbindungen der Formel (I), in denen R₁ und R₂ Wasserstoff bedeuten, 2,4-Diamino-6-(2',4'-dichlorphenoxy)pyrimidin-3-N-oxid oder 2,4-Diamino-6-chlorpyrimidin-3-N-oxid mit einem Alkoholat RO⁻ Y⁺ reagieren läßt, worin R die oben angegebene Bedeutung hat und Y ein Alkalikation ist,
(b) zum Erhalt einer Verbindung der Formel (I), worin R₁ und R₂ eine Carbamoylgruppe bedeuten, das entsprechende 2,4-Diamino-6-haloalkoxypyrimidin-3-N-oxid-Derivat mit einem Carbamoylchlorid in einem polaren Lösungsmittel bei einer Temperatur von 0 bis 100°C reagieren läßt,
(c) zum Erhalt einer Verbindung der Formel (I), worin R₁ und R₂ Alkoxycarbonylgruppen bedeuten, das entsprechende 2,4-Diamino-6-haloalkoxypyrimidin-3-N-oxid-Derivat mit einem Überschuß an Chlorameisensäureester in einem polaren aprotischen Lösungsmittel bei einer Temperatur von 0 bis 50°C reagieren läßt,
(d) zum Erhalt einer Verbindung der Formel (I), worin R₁ und R₂ Acylgruppen bedeuten, das entsprechende 2,4-Diamino-6-haloalkoxypyrimidin-3-N-oxid-Derivat mit einem Säurechlorid oder -anhydrid in einem polaren aprotischen Lösungsmittel bei einer Temperatur von 0 bis 50°C reagieren läßt.

2. Zusammensetzung zur topischen Aufbringung,
dadurch **gekennzeichnet,** daß
sie in einem physiologisch verträglichen Milieu mindestens eine in jedem der Ansprüche 1 bis 3 definierte Verbindung der Formel (I) enthält.

3. Zusammensetzung gemäß Anspruch 2,
dadurch **gekennzeichnet,** daß
sie zu ihrer pharmazeutischen Anwendung in Form einer Salbe, Tinktur, Creme, Pommade, eines Pulvers, Tupfers, getränkten Tampons, einer Lösung, Emulsion, bläschenartigen Emulsion, Lotion, eines Gels, eines Spray oder einer wasserfreien oder wässrigen Suspension vorliegt und mindestens eine in jedem der Ansprüche 1 bis 3 definierte Verbindung der Formel (I) enthält.

4. Zusammensetzung gemäß Anspruch 2 oder 3,
dadurch **gekennzeichnet,** daß
die Verbindungen der Formel (I) in Konzentrationen von 0,1 bis 20 und insbesondere von 0,2 bis 10 Gew.%, bezogen auf Gesamtgewicht der Zusammensetzung, vorhanden sind.

5. Zusammensetzung gemäß Anspruch 2,
dadurch **gekennzeichnet,** daß
sie in Form einer Lotion, eines Gels, einer Seife, eines Shampoo, Aerosols oder Schaums vorliegt und, in einem auf dem kosmetischen Plan zulässigen Trägermedium, mindestens eine in Anspruch 1 definierte Verbindung in einer Konzentration von 0,01 bis 15 Gew.% enthält.

6. Zusammensetzung gemäß jedem der Ansprüche 2 bis 5,
dadurch **gekennzeichnet,** daß
sie, zusätzlich zu hydratisierenden Mitteln, antiseborrheische Mittel enthält.

7. Zusammensetzung gemäß jedem der Ansprüche 2 bis 6,
dadurch **gekennzeichnet,** daß
sie zusätzlich Nikotinsäureester, steroide oder nichtsteroide entzündungshemmende Mittel, Retinoide, antibakterielle Mittel, Calcium-Antagonisten, Hormone, antiandrogene Mittel, Abfangmittel von OH-Radikalen enthält.

8. Zusammensetzung gemäß jedem der Ansprüche 2 bis 7,
dadurch **gekennzeichnet,** daß
sie zusätzlich Verbindungen enthält, ausgewählt aus Diazoxid, Spiroxason, Phospholipiden, Linolen- und Linolsäuren, Salicylsäure und ihren derivaten, Hydroxycarboxyl- oder Ketocarboxylsäuren, deren Estern, aus Lactonen und deren entsprechenden Salzen, Anthralin oder 1,8,9-Trihydroxyanthracen, Karotinoiden, Eicosa-5,8,11,14-tetrain-, Eicosa-5,8-11-triinsäuren, deren Estern und Amiden.

9. Zusammensetzung gemäß jedem der Ansprüche 4 bis 8
dadurch **gekennzeichnet,** daß
das physiologisch zulässige Milieu aus Wasser, einer Mischung aus Wasser und einem oder mehreren organischen Lösungsmittel(n) oder aus einer Mischung organischer Lösungsmittel zusammengesetzt ist, wobei die organischen Lösungsmittel pharmazeutisch oder kosmetisch zulässig sind.

10. Zusammensetzung gemäß Anspruch 9,
dadurch **gekennzeichnet,** daß
die Lösungsmittel aus C₁₋₄-Niedrigalkoholen, Alkylenglycolen, Alkylethern vom Mono- und Dialkylenglycol ausgewählt sind.

11. Zusammensetzung gemäß jedem der Ansprüche 4 bis 10,
dadurch **gekennzeichnet,** daß
das physiologisch zulässige Milieu mit verdickenden und/oder gelierenden Mitteln verdickt ist und Konservierungs-, Stabilisierungs, pH-Regulierungs, Modifizierungsmittel des osmotischen Drucks, Emulgiermittel Filterstoffe für UV-A und UV-B, Antioxidantien enthält.

12. Zusammensetzung gemäß jedem der Ansprüche 4 bis 11,
dadurch **gekennzeichnet,** daß
sie auch oberflächenaktive Mittel enthält, die aus nichtionischen und amphoteren oberflächenaktiven Mitteln ausgewählt sind.

13. Verfahren zur kosmetischen Behandlung der Haare oder der behaarten Haut,
dadurch **gekennzeichnet,** daß
man die in jedem der Ansprüche 4 bis 12 definierte Zusammensetzung zur Anwendung bringt.

14. Verwendung der gemäß jedem der Ansprüche 4 bis 12 definierte Zusammensetzung zur Herstellung eines Medikaments zur therapeutischen Behandlung des Ausfalls der Haare.

15. Verfahren zur Herstellung einer Zusammensetzung zur topischen Aufbringung,
dadurch **gekennzeichnet,** daß
man in ein physiologisch zulässiges Milieu mindestens eine in Anspruch 1 definierte Verbindung der Formel (I) einbringt.
